Europäisches Patentamt

European Patent Office

Office européen des brevets

(19)

(11) **EP 1 201 760 A1**

(12) **EUROPEAN PATENT APPLICATION**

(43) Date of publication:
**02.05.2002 Bulletin 2002/18**

(51) Int Cl.⁷: **C12N 15/86**, C07K 14/47,
C12N 5/10, A61K 48/00,
A61K 39/00

(21) Application number: **00123687.6**

(22) Date of filing: **30.10.2000**

(84) Designated Contracting States:
**AT BE CH CY DE DK ES FI FR GB GR IE IT LI LU
MC NL PT SE**
Designated Extension States:
**AL LT LV MK RO SI**

(71) Applicant: **ARTEMIS Pharmaceuticals GmbH**
**51063 Köln (DE)**

(72) Inventors:
• **Schuler, Gerold**
  **Dermatologische Universitätsklinik**
  **91052 Erlangen (DE)**
• **Hobom, Gerd**
  **79106 Freiburg (DE)**
• **Steinkasserer, Alexander**
  **Dermatologische Uniklinik**
  **91052 Erlangen (DE)**

• **Strobel, Isolde**
  **Dermatologische Universitätsklinik**
  **91052 Erlangen (DE)**
• **Grassmann, Ralph**
  **90480 Nürnberg (DE)**

(74) Representative:
**Helbing, Jörg, Dr. Dipl.-Chem. et al**
**Patentanwälte**
**von Kreisler-Selting-Werner,**
**Postfach 10 22 41**
**50462 Köln (DE)**

Remarks:
The sequence listing, which is published as annex
to the application documents, was filed after the date
of filing. The applicant has declared that it does not
include matter which goes beyond the content of the
application as filed.

(54) **Influenza virus vector for human dendritic cells**

(57)    The present invention relates to a method for the expression of tumour-associated antigens or virus associated antigens by dendritic cells, dendritic cells obtainable by said method and their use for treating tumours or virus infections.

**EP 1 201 760 A1**

Annex to the application documents-subsequently filed sequences listing

```
                                    SEQUENCE LISTING

            <110> ARTEMIS PHARMACEUTICALS GmbH

            <120> Influenza Virus Vector for Human Dendritic Cells

            <130> 002741ep/JH/ml

            <140> 00123687.6
            <141> 2000-10-30

            <160> 26

            <170> PatentIn Ver. 2.1


            <210> 1
            <211> 12
            <212> RNA
            <213> Influenza A virus

            <400> 1
            ccugcuuuug cu                                               12


            <210> 2
            <211> 12
            <212> RNA
            <213> Influenza B virus

            <400> 2
            nnygcuucug cu                                               12


            <210> 3
            <211> 12
            <212> RNA
            <213> Influenza C virus

            <400> 3
            ccugcuucug cu                                               12


            <210> 4
            <211> 12
            <212> RNA
            <213> Artificial Sequence

            <220>
            <223> Description of Artificial Sequence: Modified
                  influenza A 3'-sequence

            <400> 4
            ccuguuucua cu                                               12
```

```
<210> 5
<211> 12
<212> RNA
<213> Artificial Sequence

<220>
<223> Description of Artificial Sequence: Modified
     influenza A 3'-sequence

<400> 5
ccugguucuc cu                                              12


<210> 6
<211> 13
<212> RNA
<213> Influenza A virus

<400> 6
aguagaaaca agg                                            13


<210> 7
<211> 13
<212> RNA
<213> Influenza B virus

<400> 7
aguagwaaca rnn                                            13


<210> 8
<211> 13
<212> RNA
<213> Influenza C virus

<400> 8
agcaguagca agr                                            13


<210> 9
<211> 13
<212> RNA
<213> Artificial Sequence

<220>
<223> Description of Artificial Sequence: Modified
     influenza A 5'-sequence

<400> 9
agaagaauca agg                                            13


<210> 10
<211> 21
<212> RNA
<213> Influenza A virus
```

```
<400> 10
aguagaaaca aggnnnuuuu u                                          21


<210> 11
<211> 21
<212> RNA
<213> Artificial Sequence

<220>
<223> Description of Artificial Sequence: Modified
      influenza A 5'-sequence (pHL1920)

<400> 11
agaagaauca aggnnnuuuu u                                          21


<210> 12
<211> 21
<212> RNA
<213> Influenza B virus

<400> 12
aguagwaaca rnnnnnuuuu u                                          21


<210> 13
<211> 19
<212> RNA
<213> Artificial Sequence

<220>
<223> Description of Artificial Sequence: Modified
      influenza C 5'-sequence

<400> 13
aguaguaaca agrguuuuu                                             19


<210> 14
<211> 15
<212> RNA
<213> Artificial Sequence

<220>
<223> Description of Artificial Sequence: Modified
      influenza A 3'-sequence (pHL1104 and pHL1920)

<400> 14
nnnccuguuu cuacu                                                 15


<210> 15
<211> 15
<212> RNA
<213> Artificial Sequence

<220>
<223> Description of Artificial Sequence: Modified
      influenza A 3'-sequence (pHL1948)
```

```
<400> 15
nnnccugguu cuccu                                              15


<210> 16
<211> 15
<212> RNA
<213> Artificial Sequence

<220>
<223> Description of Artificial Sequence: Modified
      influenza B 3' sequence

<400> 16
nnnnnyguuu cuacu                                              15


<210> 17
<211> 14
<212> RNA
<213> Artificial Sequence

<220>
<223> Description of Artificial Sequence: Modified
      influenza C 3'-sequence

<400> 17
ccccuguuuc uacu                                               14


<210> 18


<211> 27
<212> DNA
<213> Artificial Sequence

<220>
<223> Description of Artificial Sequence: Primer

<400> 18
ggagatctca tcatgcctct tgagcag                                 27


<210> 19
<211> 45
<212> DNA
<213> Artificial Sequence


<220>
<223> Description of Artificial Sequence: Primer

<400> 19
cctctagatt atatatagcg ataggtgtcc tcttccccct ctctc            45


<210> 20
```

```
<211> 20
<212> DNA
<213> Artificial Sequence

<220>
<223> Description of Artificial Sequence: Primer


<400> 20
cgggaaatcg tgcgtgacat                                               20


<210> 21
<211> 20
<212> DNA
<213> Artificial Sequence

<220>
<223> Description of Artificial Sequence: Primer

<400> 21
gaactttggg ggatgctcgc                                              20


<210> 22
<211> 21
<212> DNA
<213> Artificial Sequence

<220>
<223> Description of Artificial Sequence: Primer

<400> 22
taatacgact cactataggg c                                            21


<210> 23
<211> 21
<212> DNA
<213> Artificial Sequence

<220>
<223> Description of Artificial Sequence: Primer

<400> 23
cagcacgtgt cttgtagttc c                                            21


<210> 24
<211> 21
<212> DNA
<213> Artificial Sequence

<220>
<223> Description of Artificial Sequence: Primer

<400> 24
cagcacgtgt cttgtagttc c                                            21
```

```
<220>
<223> Description of Artificial Sequence: Primer

<400> 25
atatagcgat aggtgtcctc                                        20


<210> 26
<211> 12
<212> RNA
<213> Artificial Sequence

<220>
<223> Description of Artificial Sequence: Modified
      influenza A 3'-sequence

<400> 26
ccuguuuuua cu                                                12
```

**EP 1 201 760 A1**

## Description

The present invention relates to a method for the expression of tumour-associated antigens or virus associated antigens by dendritic cells, dendritic cells obtainable by said method and their use for treating tumours or virus infections.

## Introduction

Anti-tumor immune responses can be efficiently generated in vivo and in vitro by dendritic cells (hereinafter shortly referred to as "DC"), which have been genetically modified to express tumor-associated antigens (hereinafter shortly referred to as "TAA"). Several viral expression vectors derived from adenoviruses, retroviruses, and poxviruses have been used to transduce foreign genes into DC. These viruses contain DNA or form a DNA intermediate, which could integrate into the host genome with the risk of adverse mutations. In contrast, influenza viruses are sensitive to antiviral pharmaceuticals, fail to establish persistent infections, do not integrate their RNA genome into the chromosome and may even be genetically designed from cloned cDNA thus providing a favourable alternative. Here we show that avian influenza virus vectors very efficiently deliver TAA genes into human monocyte-derived DC. The modified DC retained their characteristic phenotypical and immunostimulatory properties. Thus the influenza virus vector represents a valuable and safe tool in the development of DC-based immunotherapy in man.

Dendritic cells (DC) are highly potent initiators of immune responses and are widely distributed throughout lymphoid and non-lymphoid tissues (R.M. Steinman, Fundamental Immunology, fourth Ed. Ed W.E. Paul. Philadelphia: Lippincott-Raven. pp. 547 (1999)). To launch immune responses, DC have to capture and to process antigens in the periphery and to present them to rare antigen-specific T cells, which they encounter after migration to lymphoid organs. DC are therefore considered as "nature's adjuvant" (J.W. Young, K. Inaba, J. Exp. Med. 183, 7-11 (1996); G. Schuler, R.M. Steinman, J. Exp. Med. 186, 1183-1187 (1997); J. Banchereau, R.M. Steinman, Nature 392, 245-252 (1998), M. Rescigno et al., Immunol. Today 20, 200-203 (1999)). Antigen uptake is accomplished by immature DC. They mature into potent T cell stimulators in response to environmental danger signals like pro-inflammatory cytokines, dsRNA or LPS. DC which are activated in this way, migrate to the lymphoid organs, develop cytoplasmic protruding veils and up-regulate a set of characteristic surface proteins. These are required for the interaction and activation of antigen-specific T cells. Among them are CD25, CD40, CD54, CD80, CD83, CD86 as well as high levels of MHC class I and class II molecules. The ability to present antigens to naive or quiescent T cells qualifies DC as a potent biological tool for cell-based immunotherapeutical strategies directed against tumors and infectious diseases (G. Schuler, R.M. Steinman, J. Exp. Med. 186, 1183-1187 (1997); J. Banchereau, R.M. Steinman, Nature 392, 245-252 (1998)). Several studies reported beneficial anti-tumor and anti-viral effects due to DC mediated vaccinations in mice (J.I. Mayordomo et al., Nat. Med. I., 1297-1302 (1995); R.C. Fields et al., Proc. Natl. Acad. Sci., 95, 9482-9487 (1998); B. Ludewig et al., J. Vorl. 72, 3812-3818 (1998)). Recently immunization with peptide- and/or tumor lysate-pulsed DC has also been reported in humans (F.J. Hsu et al., Nat. Med. 2, 52-58 (1996); G. Murphy et al., Protstate 29, 371-380 (1996); F.O. Nestle et al., Nat. Med. 4, 328-332 (1998); M.V. Dhodapkar et al., J. Clin. Invest. 104, 173-180 (1999); M. Marchand et al., Int. J. Cancer 80, 219-230 (1999); B. Thurner et al., J. Exp. Med. 190, 1669-1678 (1999)). Although these data are quite promising, the peptide-loading strategy has several disadvantages, including HLA restrictions and the fact that only a limited number of tumor-specific peptides are known.

## Summary of the Invention

It was found that influenza virus vectors can be used to transduce TAA genes. These viruses abortively infect DC without interfering with their antigen presenting capacity. In contrast to other viruses used for DC transduction, influenza viruses can be efficiently controlled by antiviral pharmaceuticals, lack to integrate into host chromosomes and fail to establish persistent infections.The invention thus provides

(1) a method for the expression of one or more tumour-associated antigens (TAA) or virus associated antigens (VAA) by dendritic cells comprising

(a) preparing a recombinant influenza virus containing a nucleotide sequence coding for the TAA or VAA, and
(b) infecting, preferably abortively infecting, dendritic cells with the recombinant influenza virus obtained in step (a);

(2) a preferred embodiment of (1) above, wherein the segment containing the nucleotide coding for the TAA or VAA contains terminal viral RNA sequences which have been modified by nucleotide substitutions in up to five positions, resulting in improved transcription rates of both the vRNA promoter as well as the cRNA promoter as present in the complementary sequences;

(3) dendritic cells expressing one or more TAA or VAA which are obtainable according to the method of (1) or (2) above;

(4) a pharmaceutical composition comprising dendritic cells of (3) above or a mixture of dendritic of (3) above;

(5) the use of dendritic cells of (3) above for preparing a medicament for tumour therapy or antiviral therapy; and

(6) a method for treating tumours or virus infections in a mammal comprising administering the mammal dendritic cells of (3) above.

Vaccination with dendritic cells presenting tumor antigens will induce a potent primary immune response or amplify existing cytotoxic antitumor T cell responses. Therefore, tumor antigens most are suitable for immunotherapy are, ideally, strictly tumor specific, or at least the immune response and should have tumoral specificity. Many tumor antigens, however, are shared with normal cells and overexpressed in the tumor. This implies that an immune response could potentially be harmful, if an immune response to self-antigens occurs causing autoimmunity. The technology for DC vaccines shall thus result in an immune response of sufficient quality and magnitude of tumor-specific T cell responses.

Tumor-specific antigens are rare. However, a growing family of testicular antigens has been identified that are aberrantly expressed in a significant proportion of tumors of various histological types and -in addition- only in testis cells. These antigens, called cancer/testis antigens, discovered by scientists of the Ludwig Institute for Cancer Research (LICR) in Brussels and New York, and their collaborators, should ensure strict tumoral specifcity of the immune responses as the germ line cells do not express MHC-I molecules.

These antigens, for example the MAGE-, GAGE- and BAGE-families, NY-ESO-I or HOM-MEL-40 (aka SSX-2)are thus prime candidates for the DC-based antitumor vaccines when part of a potent dendritic cell vaccine based on influenza virus-mediated gene transfer.These antigens have the required selectivity for a flu-vector based DC vaccine, can most likely be readily incorporated into the recombinant virus, and are able to induce cellular immune responses. Peptides derived from MAGE-A3 have been ?pulsed unto" dendritic cells, by Schuler and coworkers and the vaccine was found to induce specific CTL. This study could serve as an historical reference.

Furthermore, the number of tumor antigens suitable for potent therapeutic vaccines is still limited and a search for novel tumor antigens, as well as viral antigens, seems warranted. The influenza virus vector system is suitable for antigen discovery (see above). Co-expression of said antigens with LICR antigens is an important option for widening the vaccine spectrum.

In experiments it was shown that genetically modified DC, which express tumour-associated antigens can efficiently induce anti-tumour immunity and thus have a high potential as tools in cancer therapy. The gene delivery is most efficiently achieved by viral vectors. Genes encoding a melanoma derived TAA, such as MAGE-3 or the green fluorescence protein (GFP) were introduced into a high-expression avian influenza virus vector. Monocyte-derived mature DC infected by these recombinants efficiently produced GFP or MAGE-3. More than 90 % of the infected DC can express a transduced gene. Importantly, these transduced DC retained their characteristic phenotype, their potent allogeneic T cell stimulatory capacity and were able to stimulate MAGE-3 specific CD8$^+$ cytotoxic T cells. Thus influenza virus vectors provide a highly efficient gene delivery system in order to transduce human DC with TAA, which consequently stimulate TAA specific T cells.

**Description of the Figures**

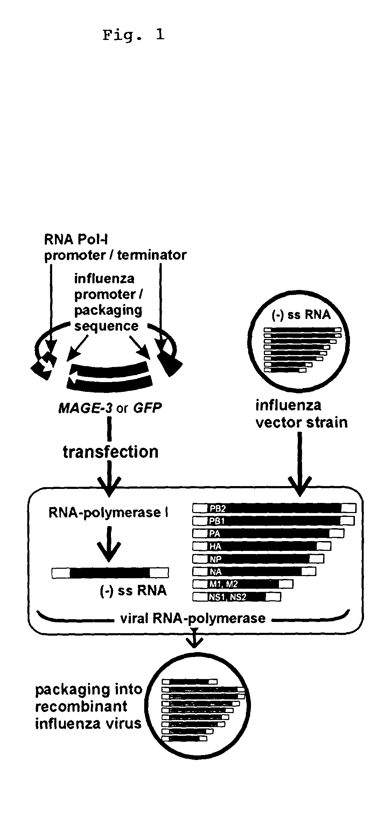

Fig.1: Insertion of the MAGE-3 gene into recombinant influenza viruses:

A plasmid containing the MAGE-3 (or GFP) open reading frame flanked by influenza virus promoter packaging signals and RNA-polymerase I control sequences was transfected into 293T cells. These cells were infected with influenza viruses. The heterologous cDNA sequences are transcribed by the cellular RNA polymerase I in virus RNA-(vRNA-) like molecules, which are subsequently amplified by the viral polymerase. The heterologous vRNA and the eight homologous influenza virus genomic RNAs are all co-packaged into virus particles.

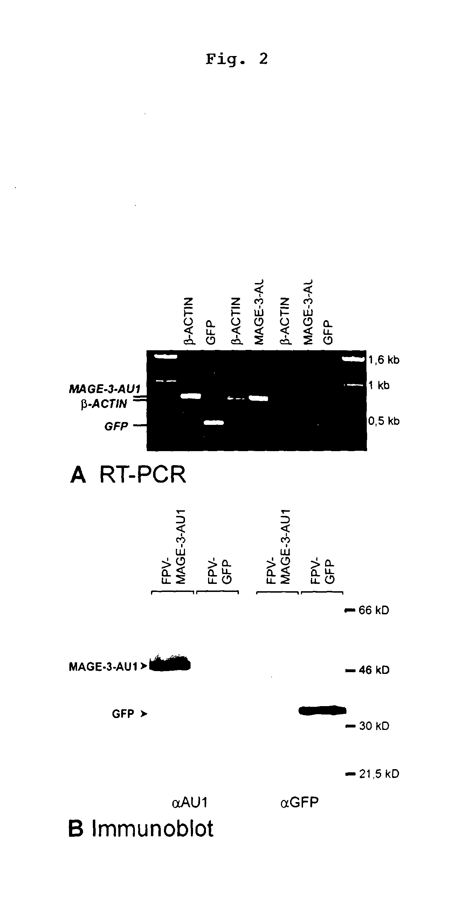

Fig. 2: High level expression of heterologous proteins from recombinant influenza viruses:

MDCK cells were infected with recombinant influenza virus stocks expressing MAGE3-AU1 (FPV-MAGE3-AU1) or GFP (FPV-GFP). (A) Heterologous RNA produced by the recombinant viruses was detected by RT-PCR with primers specific for MAGE-3 and GFP, respectively. The β-actin RT-PCR served as an internal control. The PCR product were detected in ethidium bromide stained agarose gels. (B) Proteins extracted from infected cells were analyzed by immunoblot using specific antibodies directed against the AU-1 sequence tag of MAGE3-AU1(left panel). The corresponding bands were visualized by a secondary antibody conjugated with horseradish peroxidase and an enhanced chemoluminescence (ECL) assay. The same blot was reprobed using antibodies against GFP (right panel).

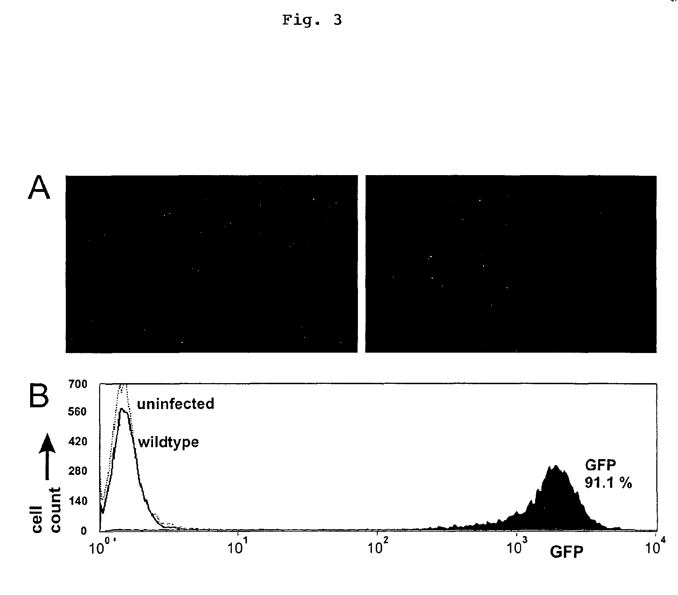

Fig. 3: Efficient gene transfer by recombinant influenza virus into human DC:

Mature human dendritic cells were infected with the recombinant influenza virus encoding GFP (FPV-GFP) at a MOI = 1. (A) After 8 hours cells expressing GFP were detected by UV-fluorescence microscopy (right panel). The left panel shows the same detail in phase contrast microscopy. (B) Quantification of the transduction rate by FACS analysis: More than 90% of the cells were found to express GFP. Uninfected and FPV wildtype infected DC were used as controls.

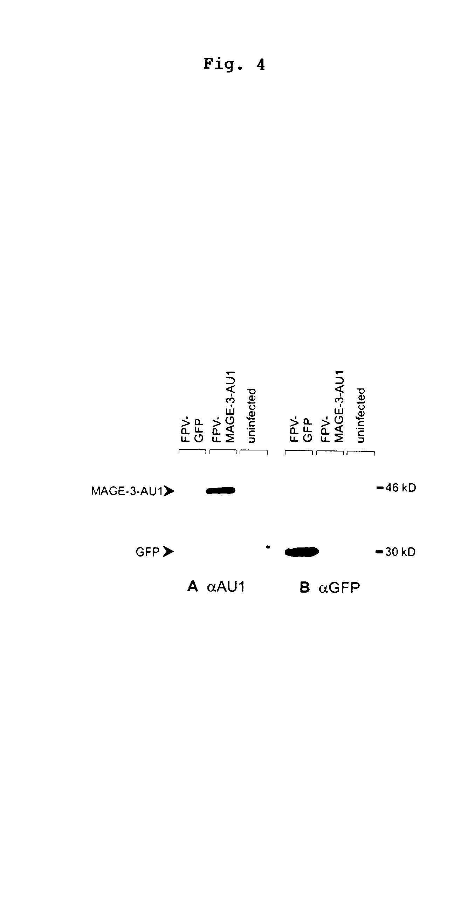

Fig. 4: Expression of heterologous MAGE-3 protein from recombinant influenza virus transduced human DC:

Mature human DC were infected (MoI = 1) with the influenza virus recombinants FPV-MAGE3-AU1 (expressing MAGE-3) or FPV-GFP (expressing GFP). After 24 hours, proteins were extracted, separated and analyzed by the immunoblot technique. (A) To detect the MAGE3-AU1 fusion protein the blot was incubated with specific antibodies to the AU-1 sequence tag. The corresponding band was visualized by a secondary antibody conjugated with horseradish peroxidase and an enhanced chemiluminescence (ECL) assay. (B) To detect GFP the same blot was reprobed using specific antibodies.

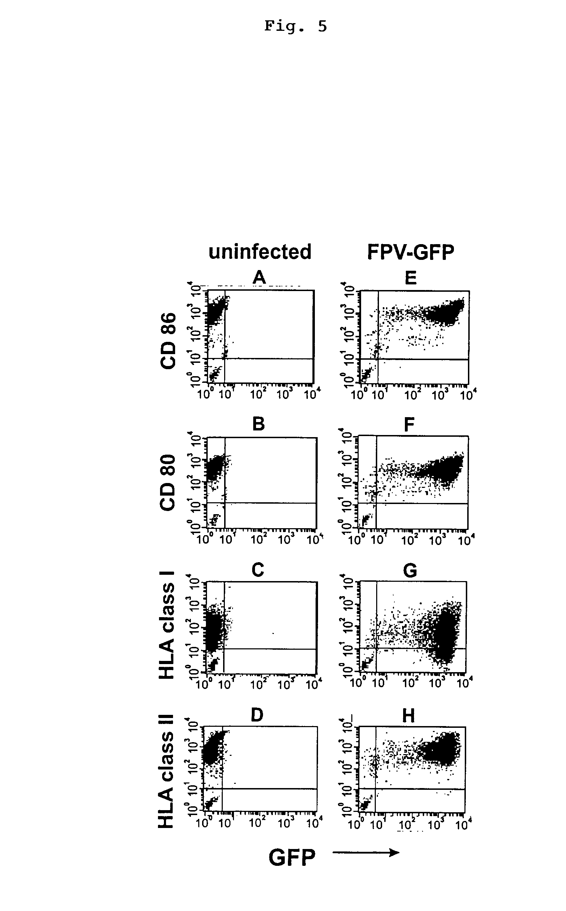

Fig. 5: Unchanged DC surface marker expression after gene transfer by recombinant influenza virus:

Mature DC were infected (MoI = 1) with GFP-encoding recombinant influenza virus (FPV-GFP) or left uninfected. After 24h, cells were counter-stained using antibodies directed CD86, CD80, HLA class I and class II and secondary PE-conjugated goat anti-mouse IgG F(ab')$_2$ fragments. The expression of cell surface markers and GFP was quantified by flow cytometry. Percentages of cells in the quadrants are indicated.

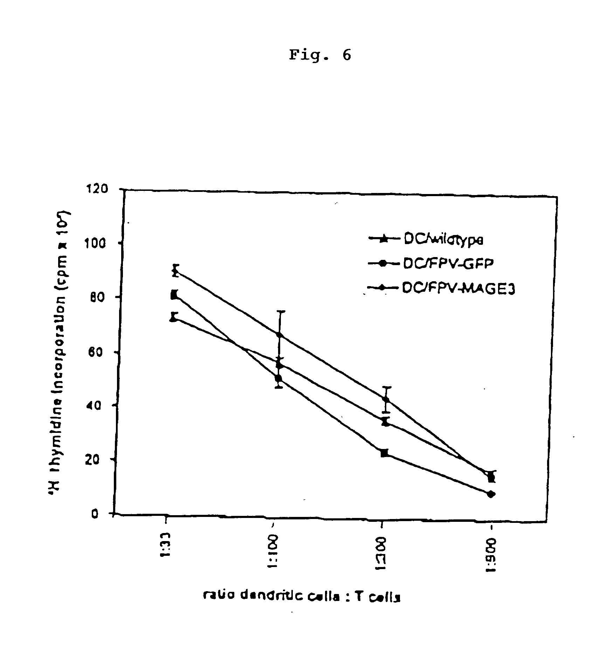

Fig. 6: Influenza virus transduced DC are potent T cell stimulators:

The allo-stimulatory capacity of DC infected with FPV-GFP(Z), with FPV-MAGE3-AU1(,) or with the FPV wild type strain(•) was tested in a MLR assay. As indicated, graded DC (6000-220) were co-cultivated with 2x10$^5$ T cells. After four days, cells were pulsed with [$^3$H]-thymidine for 16 hours and incorporation of radioactivity was determined. Shown are the mean counts $\pm$ SEM of triplicates. Counts for T cells alone or DC alone were always less than 1000 cpm.

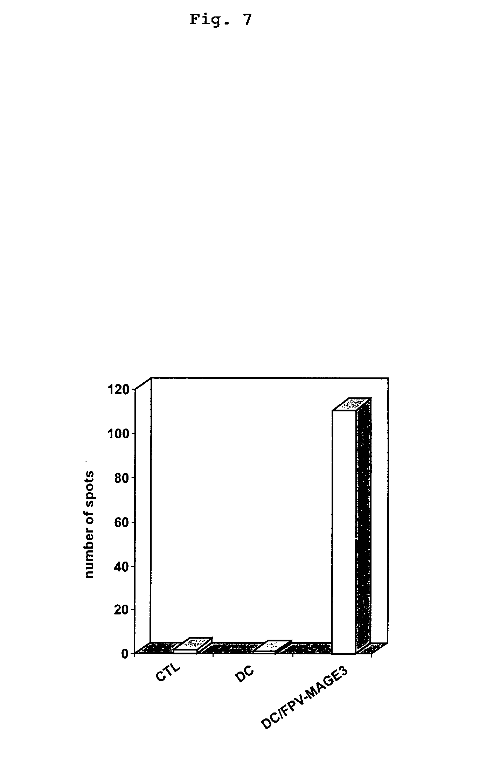

Fig.7: Detection of MHC-I/IMP complexes on the transduced DC by a specific CTL clone:

Uninfected or FPV-MAGE3-AU1 infected DC (7.5 x 10$^4$) were co-cultured with an IMP-specific CTL clone ( 5 x 10$^3$) for 18 hours. The capacity of the CTL clone to release IFN-( was immunochemically visualized by the ELISPOT-technique. Results are shown as mean spot numbers $\pm$ SEM of triplicates.

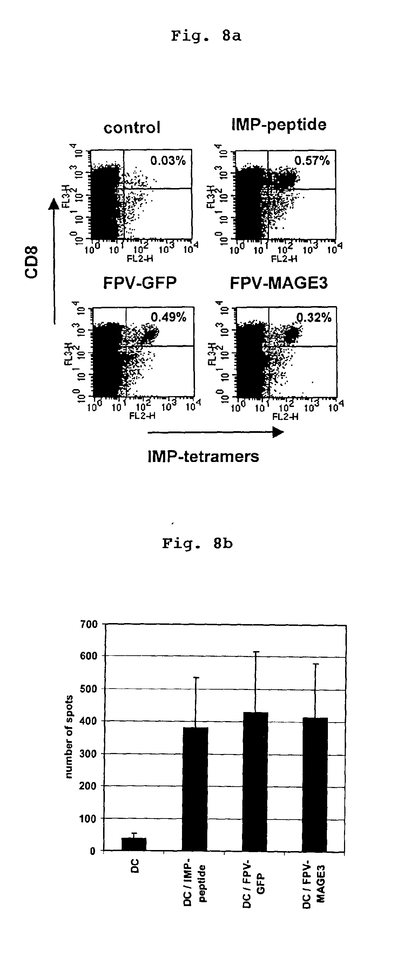

Fig. 8: Expansion of CD8$^+$ memory T cells in the absence of exogenous cytokines by transduced DC:

To demonstrate that DC infected with recombinant influenza virus are able to stimulate influenza virus-specific CTL, autologous T cells (4 x 10$^6$) were co-cultured with 0.2 x 10$^6$ DC for seven days without addition of exogenous cytokines. (A) Subsequently the IMP-specific CTLs were stained with tetrameric MHC-I/IMP complexes at 37°C and counterstained with labelled CD8 mAb at 4°C. The IMP-specific CD8$^+$ T cells were identified by FACS analyses (upper right sector). The dot plots show T cells co-cultured with uninfected DC (control), IMP peptide-pulsed DC (IMP-peptide), FPV-GFP infected DC (FPV-GFP) or with FPV-MAGE3-AU1 infected DC (FPV-MAGE3).(B) Demonstration of IFN-( release from IMP-specific CTLs. The CTL activity upon stimulation with IMP peptide (1μM) was assessed by ELISPOT-assay with 0.5 x 10$^6$ T cells per well. Cells were cultured for 18 hours and IFN-( release from individual T cells was immunochemically visualized. Shown are means $\pm$ SEM of triplicates

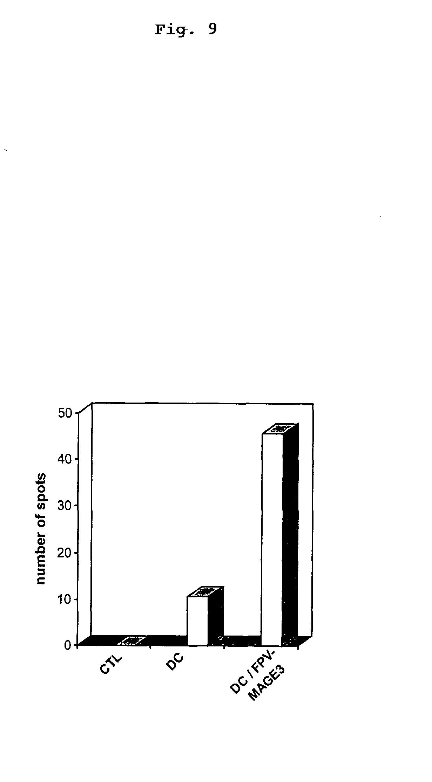

Fig. 9: Stimulation of MAGE-3 specific CTL by transduced DC:

Uninfected or FPV-MAGE3-AU1 infected DC (7.5 x10$^4$), were co-cultured with MAGE-3 specific CTL clone (5 x 10$^3$) for 18 hours. The capacity of transduced DC to present antigen was evaluated by IFN-g secretion of individual CTL using the ELISPOT-technique. Results are the mean of three experiments $\pm$ SEM.

**Detailed Description of the Invention**

The strategy to genetically modify DC by genes coding for full-size tumor antigens has several advantages over pulsing DC with whole tumor lysates or tumor peptides: The heterologous proteins expressed from the introduced genes are endogenously processed, loaded onto MHC class I molecules (Y. Yang et al., J. Virol. 69, 2004-2015 (1995); C.A. Mack et al., Hum. Gene Ther. 8, 99-109, (1997)) and presented to T lymphocytes. Thus genetically modified DC have the potential to present previously unknown epitopes in association with different MHC molecules. Therefore, new strategies for effective genetic modifications are needed in order to generate new DC based anti-tumor vaccines. DC can be generated in vitro from cultured human monocytes (N. Romani et al., J. Exp. Med. 180, 83-93 (1994); F. Sallusto, A. Lanzavecchia, J. Exp. Med. 179, 1109-1118 (1994); A. Bender et al., J. Immunol. Methods 196, 121-135 (1996); N. Romani et al., J. Immunol. Methods 196, 137-151 (1996)) even in large numbers from leukapheresis products for clinical use (B. Thurner et al., J. Exp. Med. 190, 1669-1678 (1999)). Transduction of these DC with tumor-associated or viral antigens leads to the presentation of these antigens via MHC class I molecules.

Viral expression vectors derived from adenoviruses, retroviruses, and poxviruses have been reported to efficiently transduce foreign genes into DC (M.E. Reeves et al., Cancer Res. 56, 5672-5677 (1996); W. Song et al., J. Exp. Med. 186, 1247-1256 (1997); M. Di Nicola et al., Cancer Gene Ther. 5, 350-356 (1998); M. Subklewe et al, Blood 94, 1372-1381 (1999); L. Zhong et al., Eur. J. Immunol. 29, 964-972 (1999); S. Yang et al., J. Immunol. 164, 4204-4211 (2000)). These viruses contain DNA or have an DNA replication intermediate, which could integrate into the host chromosomal sequences thus posing the risk of adverse mutations. This is impossible with negative-strand RNA viruses as influenza viruses. Interestingly, DC infected by influenza viruses are strong stimulators of human CD8[+] T cells (N. Bhardwaj et al., J. Clin. Invest. 94, 797-807 (1994); A. Bender et al., J. Exp. Med. 182, 1663-1671 (1995) and Immunology 198, 552-567 (1998)). Furthermore, infection by influenza virus results in the maturation of immature DC and to increased antigen presentation and T cell stimulatory capacity (M. Cella et al., J. Exp. Med. 189, 821-829 (1999)). Therefore gene transfer into DC by influenza virus vectors represents a promising approach to induce anti-tumor or anti-viral immune response. The generation of recombinant influenza viruses was hampered for a long time by the fact that the virus has a segmented RNA genome. The development of the RNA polymerase I technique allows the generation of recombinant viruses with additional genomic segments capable of expressing complete heterologous genes (G. Neumann et al., Virology 202, 477-479 (1994)), which was built around *in vivo* synthesis of recombinant vRNA molecules by cellular RNA polymerase I transcription of the respective template cDNA constructs, modified terminal viral RNA sequences (hereinafter "promoter-up mutations" or promoter-up variants") have been designed by nucleotide substitutions (Neumann and Hobom, Mutational analysis of influenza virus promoter elements *in vivo,* J. Gen. Virol. 76, 1709-1717 (1995); WO 96/10641). The above promoter-up variants carry up to five nucleotide substitutions (in promoter-up variant 1920; see Flick and Hobom, J. Gen. Virol. 80, 2565-2572 (1999)). When these promoter-up variants are attached to a recombinant ninth vRNA segment its increased transcription and amplification rate will not only compensate for the losses suffered spontaneously, but even cause accumulation of the foreign vRNA segment during simple viral passaging, in the absence of any selection.

As set forth in embodiment (2) above, a preferred method of the invention is where the recombinant virus contains terminal viral RNA sequences, which are active as promoter signal, have been modified by nucleotide substitution in up to 5 positions, resulting in improved transcription rates (of both the vRNA promoter and in the cRNA promoter as present in the complentary sequence) as well as enhanced replication and/or expression rates relative to the wild-type sequence. Said modified terminal viral RNA sequences differ from the wild-type sequence in that in said tandem vRNA segment the 12 nucleotide conserved influenza 3' terminal sequence has been modified by replacement of one to three nucleotides occurring in said sequence at positions 3, 5 and 8 relative to the 3' end by other nucleotides provided that the nucleotides introduced in positions 3 and 8 are forming a base pair (i.e., if the nucleotide position 3 is G, than that in position 8 is C; if the nucleotide in position 3 is C, than that in position 8 is G; etc.).

The 3' conserved regions of the wild-type influenza virus have the following sequences:

Influenza A: (5')-CCUGCUUUUGCU-3'

Influenza B: (5')-NN(C/U)GCUUCUGCU-3'

Influenza C: (5')-CCUGCUUCUGCU-3'.

Moreover, the 13 nucleotide conserved influenza 5'-terminal sequence may be modified by replacement of one or two nucleotides occurring in said sequence as positions 3 and 8 by other nucleotides, again provided that the introduced nucleotides are forming a base pair. The 5' conserved regions of the wild-type influenza virus have the following sequences:

<div align="center">

### Influenza A: 5'-AGUAGAAACAAGG

### Influenza B: 5'-AGUAG(A/U)AACA(A/G)NN

### Influenza C: 5'-AGCAGUAGCAAG(G/A):

</div>

Preferred influenza viruses of the invention are those wherein in the 3' conserved region the replacements G3A and C8U have been performed, more preferred are those where also the replacement U5C has been performed (the above mutations are annotated relative to the 3' end; such counting from the 3' end is also indicated by a line on top of the digit, e.g., G $\bar{3}$A). Another preferred influenza virus mutant comprises the 3'-terminal nucleotide sequence G3C, U5C and C8G (relative to the 3' end) resulting in the following 3' terminal nucleotide sequence (5')-CCUGGUUCUCCU-3'. Among the influenza viruses defined hereinbefore those having a 3'-terminal nucleotide sequence of (5') -CCUGUUUCUACU-3' are most preferred. In case of an influenza A virus the segment may further have the modifications U3A and A8U in its 5' terminal sequence, in case of influenza C it may have the modifications C3U and G8A in its 5' terminal sequence. The most preferred influenza viruses of the present invention comprise the following general structures:

#### Influenza A (mutant pHL1102):

5'-AGUAGAAACAAGGNNNU$_{5\text{-}6}$..(880-2300 ntds)..N'N'N'CCUG<u>U</u>UUUU<u>A</u>CU-3'

#### Influenza A (mutant pHL1104):

5'-AGUAGAAACAAGGNNNU$_{5\text{-}6}$..(880-2300 ntds)..N'N'N'CCUG<u>U</u>UU<u>CU</u><u>A</u>CU-3'

#### Influenza A (mutant pHL1920):

5'-AG<u>A</u>AGAA<u>U</u>CAAGGNNNU$_{5\text{-}6}$..(880-2300 ntds)..N'N'N'CCUG<u>U</u>UU<u>CU</u><u>A</u>CU-3'

#### Influenza A (mutant pHL1948):

5'-AGUAGAAACAAGGNNNU$_{5\text{-}6}$..(880-2300 ntds)..N'N'N'CCUG<u>G</u>UU<u>CU</u><u>C</u>CU-3'

#### Influenza B:

5'-AGUAG(A/U)AACA(A/G)NNNNNU$_{5\text{-}6}$..(880-2300 ntds)..N'N'N'N'N'(C/U)G<u>U</u>UUCU<u>A</u>CU-3'

#### Influenza C:

5'-AG<u>U</u>AGUA<u>A</u>CAAG(G/A)GU$_{5\text{-}6}$..(880-2300 ntds)..CCCCUG<u>U</u>UUCU<u>A</u>CU-3'

In the above structures the variables are defined as follows:

(1) Underlined and enlarged letters show the required mutations relative to the wild-type sequence for preparing a promoter mutant with enhanced properties;
(2) enlarged A in position 10 in the 5'-part of the sequence: unpaired A residue, bulge-forming;

(3) (A/G) in one position: different isolates or single segments with variable sequence at the respective position, which are functionally interchangeable;

(4) N and N': positions undefined, but base-paired relative to each other because of complementarity between the 5' and 3' termini, different among the 8 segments, but constant for each segment throughout all viral isolates;

(5) (880-2300 ntds): the lengths of the viral RNA segments, in case of segments with foreign genes increased up to 4,000 nucleotides.

Here is described a new transduction system for DC based on a recombinant avian influenza virus vector. Using this vector more than 90 % of the DC expressed the marker green fluorescent protein (GFP) and similarly the tumor-associated antigen MAGE-3 was efficiently expressed. These transduced DC retained their characteristic phenotype and their potent T cell stimulatory capacity. Furthermore, they were also able to stimulate MAGE-3 specific CD8[+] memory T cells as well as CTL clones. Expression of heterologous genes in dendritic cells (DC) is a powerful strategy to elicit strong immune reactions against tumor antigens. Here we report that influenza viruses are capable to efficiently introduce and express foreign proteins such as tumor associated antigens (TAA) like MAGE-3 or virus associated antigens (VAA) in functional monocyte-derived human DC, thus providing a potential tool for immune therapy of malignant melanoma and other tumors.

Suitable TAAs in accordance with the present invention are those known in the art, including, but not limited to, TAAs from the class of cancer/testis antigens. Preferably the TAA is MAGE-3, NY-ESO-1 or HOM-MEL-40.

Suitable VAAs in accordance with the present invention are those known in the art including, but not limited to, VAAs selected from a Herpes simplex virus immediate early or early protein, a human papilloma virus-antigen, preferably $L_1$, $E_5$ or $E_6$, a Human Immunodeficiency Virus protein, preferably tat, rev or gag, or a Hepatitis C virus protein known to elicit T cell response in a mammal.

The TAA or VAA may also be a fusion protein, preferably a fusion protein comprising entirely or partially the TAA or VAA proteins listed above. Viral vectors represent a very powerful method to deliver heterologous genes into primary cells. Several viral vector systems have been described that are capable to introduce foreign genes into DC. Adenoviral vectors have been reported to efficiently transduce human CD34[+]-derived (M. Bregni et al., Gene Ther. 5, 465-472 (1998)) as well as monocyte-derived DC (A.B. Dietz, P.S. Vuk, Blood 91, 392-398 (1998); L. Zhong et al., Eur. J. Immunol. 29, 964-972 (1999)). Retrovirus vectors have been shown to transduce proliferating human DC progenitors (J.F. Arthur et al., Cancer Gene Ther. 4, 17-25 (1997); P. Szabolcs et al., Blood 90, 2160-2167 (1997); B. Verhasselt et al., Blood 91, 431-440 (1998)). However, there are several disadvantages associated with retroviral vectors. They have relatively low transduction rates (P. Szabolcs et al., Blood 90, 2160-2167 (1997)) and the genomes of these vectors integrate into the host genome. Furthermore, non-dividing cells, like terminally differentiated, mature DC cannot be transduced. Vaccinia viruses have also been used to efficiently transfer genes into human DC. However this vector employs multiple mechanisms to evade the immune system including down modulation of the costimulatory molecule CD80 and induces apoptotic cell death in both stages of DC (J. Engelmayer et al., J. Immunol 163, 6762-6768 (1999), R. Drillien et al., Virology 268, 471-481 (2000)).

More than 90 % of mature DC expressed the GFP marker after infection with recombinant influenza virus without undergoing lysis. This is comparable with the very high transduction rate reported for recombinant GFP adenoviral vectors (L. Zhong et al., Eur. J. Immunol. 29, 964-972 (1999)). The DC transfected with the novel vector did not reveal changes in their typical surface marker expression with the exception of a moderate down-modulation of CD83. This is most probably a strain specific feature of FPV, since infection with another influenza virus strain (PR-8) had no impact on the typical surface expression of this protein (M. Cella et al., J. Exp. Med. 189, 821-829 (1999)). The DC transfected with the novel vector express viral and heterologous protein and generate MHC-I peptide complexes as shown by using CTL clones in conjunction with interferon-( ELISPOT. We also demonstrated immunogenicity of the transfected DC. They were able to expand influenza virus matrix protein-specific memory T cells in the absence of exogenous cytokines. This is a unique feature of mature potent immunostimulatory DC. Furthermore, infected DC were still very potent allogeneic T cell stimulators and were also able to stimulate MAGE-3 specific CTL clones. Therefore, mature DC which have been subjected to influenza vector-mediated heterologous gene transfer are potent antigen presenting cells.

The features of this influenza virus vector suggest that it could be developed into a safe vector system useful also for the application in humans. In contrast to other viral vector systems, influenza virus does not contain DNA nor does its replication depend on a DNA intermediate. Thus influenza virus sequences cannot integrate into genomic host sequences and cause mutations or cancer. There is no clinical indication that influenza virus infection can become persistent or chronic in humans, as has been known for retroviruses and adenoviruses (H. Hayder A. Mullbacher, Immunol. Cell. Biol. 74, 504-512 (1996)). Recombinant influenza viruse have a limited capacity to replicate in culture, thus can be considered as self-attenuating. Usually after four passages the infectivity of viral stock is drastically decreased. The self-attenuation is due to the superior capacity of the promoter variant containing heterologous segment to replicate. Thereby it prevents the incorporation of essential genomic segments into viral particles after exceeding a certain threshold. Finally, several antiviral substances including neuraminidase inhibitors are known to interfere with influenza virus propagation, providing a backup in case of an accidentally induced infection (R. Lambkin, J.S. Oxford, Textbook on Influenza, p. 487 (1998); C.R. Penn, Textbook on Influenza, pp. 477-487 (1998)).

Like other viral vector systems used for gene transfer into DC, the influenza virus vector results in the presentation of viral vector antigens in addition to the TAA. Simultaneous presentation of several antigens could result in unequal immune reactions with the activation of CTL against one or few dominant antigens. Such immunodominance in the CTL response probably results from interference between T cell responses and not from insufficient presentation of peptide epitopes (E.Z. Wolpert et al., J. Immunol. 161, 4449-4505 (1998)). Immunodominance of viral vector antigens over heterologous tumor associated antigens cannot be excluded for all MHC-I haplotypes. However, the activation of a MAGE-3-specific CTL clone by influenza virus-transduced DC strongly argues for effective presentation of the TAA. Furthermore, since dominant T cell epitopes for influenza viruses are well characterized and influenza viruses can be generated entirely from cloned DNA (G. Neumann et al., Proc. Natl. Acad. Sci., 96, 9345-9350 (1999)), it is feasible to assume that competing viral epitopes in the vector may be removed by mutagenesis. For long-term genetic modifications, the immunogenicity of the used vector might be a major obstacle, since the cellular immunity directed against influenza virus could lead to the destruction of genetically modified DC. This has been reported for instance for adenoviral vectors upon application of these vectors to elicit anti-tumor immune responses in gene therapy studies (G.P. Gao et al., J. Virol. 70, 8934-8943 (1996) and Immunology 91, 135-144 (1997); M. Christ et al., Immunol. Lett. 57, 19-25 (1997)). On the other hand, for immunotherapy studies, using short-lived genetically modified DC this cellular immunity problem might not be as severe. The presence or development of neutralizing antibodies against the viral vector might not be of major concern in an ex vivo gene transfer approach with no production of viral progeny. Influenza viruses are internalized upon DC infection, which also becomes abortive during late gene expression (A. Bender et al., Exp. Med. 182, 1663-1671 (1995) and Immunobiology 198, 552-567 (1998)). This suggests that the chances of direct exposure of complete viral surface antigens that can be recognized by host antibodies are reduced. Additional techniques to attenuate the virus are available leading to further reduction in replication (Hobom, unpublished). Since it is now possible to efficiently generate influenza A viruses entirely from cloned cDNAs (Neumann *et al.,* 1999) it is feasible to directly introduce new haemagglutinin genes which are not recognized by the immune system. In addition, influenza viruses might also be applied in combination with other viral vectors in a sequential approach to boost the immune reaction against the heterologous antigens and to minimize the reaction to the viral proteins.

In summary, the influenza virus expression vector system offers a unique combination of features which are advantageous for an ex-vivo transduction of DC. It combines high efficiency with genomic simplicity, lack of genomic integration, and the availability of a chemotherapeutic backup safety measure. Thus influenza virus vectors may be a valuable alternative in the development of genetically modified DC. To minimize the anti-vector immunization and booster the therapeutic anti-tumor immune responses, it might be alternated with other vector systems expressing the same TAA in sequential immunization protocols.

The invention is further explained by the following non-limitative experiments.

**Experiments**

1. Materials and Methods, Cell lines and viruses:

The human embryonic kidney cell line 293T and the Madin-Darby canine kidney (MDCK) cell line were cultivated in Dulbecco's modified Eagle Medium (DMEM) supplemented with 10% fetal calf serum (FCS), glutamine (0.35 mg/ml), penicillin (0.12 mg/ml) and streptomycin sulfate (0.12 mg/ml). The medium for 293T cells was additionally supplemented with G418 (500μg/ml) in order to maintain high T-Ag expression. The human melanoma cell line MZ2-MEL 43, the EBV-transformed B cell line LG-2-EBV and the HLA-A1 restricted MAGE-3-specific CTL clone LB 705 CTL 434/1 were kindly provided by P. v.d. Bruggen (Ludwig Institute for Cancer Research; Brussels, Belgium). MZ2-MEL 43 and LG-2-EBV cells were cultured in DMEM or Iscove's medium (Life Technologies, Karlsruhe, Germany) supplemented with 10 % FCS. The MAGE-3.A1 CTL clone was cultured in Iscove's medium supplemented with 10 % pooled human serum, L-arginine (116 μg/ml), L-asparagine (36 μg/ml) and gentamycin (10 μg/ml). This CTL clone was restimulated weekly with irradiated (100 Gy) MAGE-3.A1 peptide loaded MZ2-MEL 43 cells. Irradiated (100 Gy) LG-2-EBV cells were used as feeder cells. The CTL clone C9, which specifically recognizes the HLA-A2.1 restricted epitope of influenza virus matrix protein 1 (IMP) was cultured in the same medium as the MAGE-3.A1 CTL clone. The CTL clone C9 was restimulated with PHA-treated irradiated (30 Gy) PBMC as stimulator cells. Again, irradiated (100 Gy) LG-2-EBV were used as feeder cells. All cell lines and clones were maintained at 37°C in an 8 % $CO_2$ atmosphere. The avian influenza A virus (fowl plaque virus, FPV H7N1) was propagated in MDCK cells as previously described (G. Neumann, G. Hobom, J. Gen. Virol. 76, 1709-1717 (1995)). Infection at a multiplicity of infection (MoI) of $1\times10^{-5}$ to $1\times10^{-6}$ resulted in virus stocks containing approximately $1\times10^9$ plaque forming units (pfu) per ml after two days of incubation. As a measure of the virus particle content, titers of virus hemagglutination were determined using chicken erythrocytes. For the quantification of infectious particles, plaque assays and limiting dilution assays were performed on MDCK cells as described previously (A. Bender et al., J. Exp. Med. 182, 1663-1671 (1995)). To determine the fraction of viruses expressing the heterologous green fluorescent protein (GFP), limiting dilution assays were examined using a fluorescence microscope (Leica DM IRB; Leica, Bensheim, Germany).

2. Preparation of recombinant influenza virus:

Heterologous genes were introduced into influenza viruses by the application of the RNA polymerase I technique (A. Zobel et al., Nucleic Acid Res. 21, 3607-3614 (1993)). This strategy leads to the incorporation of a heterologous gene into the influenza virus particle as an additional, ninth genomic segment (pseudo-vRNA). The required RNA is transcribed from transfected plasmid DNA, which encodes the recombinant gene in combination with modified influenza virus 3' plus 5' terminal sequences (Fig.1). These constitute an influenza virus promoter-up variant and contain the viral promoter and packaging signals. For expression of the influenza virus pseudo-vRNA the plasmid contains RNA polymerase I transcription control elements, that allow the generation of uncapped, non-polyadenylated RNA with the flanking influenza virus control sequences. Influenza virus recombinants were essentially prepared as described before (G. Neumann et al., Virology 202, 477-479 (1994)). Briefly, plasmids (3 µg) encoding the heterologous viral genomic segment with a promoter-up mutation (G. Neumann, G. Hobom, J. Gen. Virol. 76, 1709-1717 (1995)) were transfected into 293T cells cultured in 6cm plates using Lipofectamin Plus (Life Technologies, Karlsruhe, Germany). After 24 hours, medium was removed from the culture and cells were inoculated with 2 ml of virus suspension (FPV Bratislava) in phosphate buffered saline (PBS) supplemented with $CaCl_2$ (0.91mM) and $MgCl_2$ (0.49mM) (=PBS++) at a MoI of 2. After 45 min, the cells were washed once with PBS++ before 3 ml of medium were added. Eight hours later, after one replication cycle, the supernatant containing the recombinant virus was harvested. To enrich for recombinant viruses, stocks were serially passaged on MDCK cells at a MoI of 2 or 3. After three passages most viruses contained the heterologous sequence as was monitored by GFP expression in limiting dilution assays. The enrichment is due to the higher replication rate of the heterologous fragment compared to the wild type segments originating from the helper virus.

3. Plasmid construction:

The plasmid pHH10 contains all *cis*-acting sequences required for the synthesis of an influenza virus genomic segment. These include the influenza virus vRNA 5' and 3' terminal sequences with promoter and packaging signal activity which are flanked by an RNA polymerase I (pol I) promoter and an RNA polymerase I terminator element. Heterologous sequences were cloned between the two parts of the influenza virus promoter in negative orientation relative to the pol I promoter, using the pBSK multiple cloning site present in this location. The coding region for MAGE-3 was generated by PCR amplification of a 983 bp fragment using plasmid pcDNA1-MAGE3 (B. Gaugler et al., J. Exp. Med. 179, 921-930 (1994)) as template (kindly provided by P. v. d. Bruggen, Brussels, Belgium). The sense primer adds a *Bgl II* site to the 5' end ( 5'- GGA GAT CTC ATC ATG CCT CTT GAG CAG -3') and the reverse primer adds an AU1 tag (P.S. Lim et al., J. Infect. Dis. 162, 1263-1269 (1990)) as well as an *Xba I* site to the 3' end (5' - CCT CTA GAT TAT ATA TAG CGA TAG GTG TCC TCT TCC CCC TCT CTC -3'). The PCR fragment was cleaved using *Bgl II* and *Xba I,* purified from agarose gel (QIAEX II gel extraction kit, Qiagen, Hilden, Germany) and cloned into pHH10 via *Bam HI* and *Xba I.* The resulting plasmid was designated pM4HH. The plasmid pHL2251 is a derivative of pHH10 containing a variant of the *Aequorea victoria* GFP gene, which will be published elsewhere.

4. RT-PCR:

Total RNA was extracted from infected MDCK cells 8 hours post infection using the High Pure RNA Isolation Kit (Roche Molecular Biochemicals, Mannheim, Germany). Subsequently, RT-PCR was performed using the Titan One Tube RT-PCR System (Roche Molecular Biochemicals, Mannheim, Germany) according to the manufacturers instructions. The PCR amplification was performed using the following conditions: 95°C for 2 min (first cycle only), 95°C for 20 s, 57°C for 20s, 72°C for 45s (21 cycles), 95°C for 20 s, 57°C for 20s, 72°C for 60s (19 cycles), 72°C for 4 min. Each RT-PCR reaction was performed in a final volume of 25µl, with 1µl of total RNA as template. The nucleotide sequences of the PCR primers used, were as follows: sense 5'-CGG GAA ATC GTG CGT GAC AT -3' and reverse 5'-GAA CTT TGG GGG ATG CTC GC -3' yielding a 712 bp beta-actin fragment; sense 5'- TAA TAC GAC TCA CTA TAG GGC -3' and reverse 5'- CAG CAC GTG TCT TGT AGT TCC -3' yielding a 397 bp GFP fragment; sense 5 - CAG CAC GTG TCT TGT AGT TCC -3' and reverse 5 '-ATA TAG CGA TAG GTG TCC TC-3' yielding a 747 bp MAGE-3-AU1 fragment.

## 5. Immunoblot:

Total cellular protein from infected MDCK cells and DC was prepared according to a freeze-thaw lysis protocol (I. Schmitt et al., J. Virol. 72, 633-640 (1998)). The lysis buffer contained 1 mM PMSF, 0.02mg/ml Aprotinin, 0.02 mg/ml Leupeptin and 2 mM DTT in 1 x TNE (Tris, NP-40, EDTA). After electrophoresis on a 12% polyacrylamide gel, proteins were transferred onto Immobilon P membrane (Millipore, Eschborn, Germany), and the blots were incubated with an anti-AU1 mouse monoclonal antibody (1:5000) (HiSS Diagnostics, Freiburg im Breisgau, Germany), washed three times and then incubated with a secondary anti-mouse antibody (1:2000) conjugated with horseradish peroxidase (Amersham Buchler, Braunschweig, Germany). GFP on immunoblots was detected with an anti-GFP rabbit polyclonal antibody (1:1000) (Clontech, Heidelberg, Germany) and a secondary anti-rabbit antibody (1:2000) also conjugated with horseradish peroxidase (Amersham Buchler, Braunschweig, Germany). Proteins recognized by these antibodies were detected using an enhanced chemoluminescence assay (Amersham Buchler, Braunschweig, Germany).

## 6. Generation and infection of human DC:

Dendritic cells were generated from CD14$^+$ human monocytes. PBMC were isolated from buffy coats of healthy HLA-typed donors by Ficoll-Hypaque density gradient centrifugation (460 x g, 25 min, 20°C). To remove platelets, PBMC were repeatedly centrifuged with decreasing g numbers. CD14$^+$ cells were positively selected using magnetic microbeads (Miltenyi, Bergisch Gladbach, Germany). As assessed by FACS-analyses, the CD14$^+$ cells were on average over 95% pure. Immunophenotypically these CD14$^+$ cells were HLA-class I$^{++}$, HLA-DR$^{++}$, CD86$^{++}$, CD40$^+$, CD83$^-$, CD80$^-$, CD25$^-$. The isolated CD14$^+$ cells were cultured at a density of 7 x 10$^6$ in a volume of 12 ml in 10 cm dishes (Falcon 3003, Becton Dickinson Heidelberg, Germany) in RPMI (BioWhittaker, Walkersville, MD, USA) supplemented with 1 % heat-inactivated AB-plasma. To start the differentiation to DC, 800 U/ml rhGM-CSF (Leukomax, Novartis, Basel, Switzerland) and 1000 U/ml rhIL-4 (Novartis Research Institute, Vienna, Austria) were added (day zero). On day three one third of the culture medium was replaced with fresh culture medium supplemented with 400 U/ml rh-GM-CSF and 500 U/ml rhIL-4. On day six the final maturation of DC was induced by adding IL-1$ (2 ng/ml) (Sigma, Deisenhofen, Germany), IL-6 (1000 U/ml) (Novartis, Basel, Switzerland), PGE2 (1 µg/ml) (Sigma) and TNF-" (10 ng/ml) (Boehringer Ingelheim Austria, Vienna, Austria) according to H. Jonuleit et al., Eur. J. Immunol. 27, 3135-3142 (1997). On day eight or nine FACS-analyses revealed the typical cell surface marker expression of mature DC: CD25$^{++}$, CD40$^{++}$, CD80$^{++}$, CD83$^{++}$, CD86$^{++}$, HLA-DR$^{++}$, HLA-class I$^{++}$, CD14$^-$. On day eight or nine mature DC (1 x 10$^6$) were infected in 1 ml serum-free medium for 1 hour at 37°C/5% CO$_2$ at a MoI of 1. Thereafter, cells were washed extensively with RPMI and cultured in 6-well-plates in RPMI supplemented with 1 % AB-plasma.

## 7. Immunophenotyping with antibodies and tetramers:

Cell surface antigens were detected by incubating cells (1 x 10$^6$/ ml) for 30 min at 4°C with monoclonal antibodies specific for CD25 (clone 2A3) from Becton Dickinson ( Heidelberg, Germany), CD14 (UCHM-1), CD40 (B-B20), CD86 (BU-63), HLA class I (W6/32), HLA-DR (DDII), all from Cymbus Biotechnology (Chandlers Ford Hants, U.K.), CD80 (MAB104), and CD83 (HB15a) both from Immunotech (Hamburg, Germany). After washing, cells were incubated with PE-conjugated goat anti-mouse IgG F(ab')$_2$ fragments (Jackson Immuno Research, West Grove, PA, USA) for 30 min at 4°C. FACS-analyses were performed on a FACScan (Becton Dickinson, Heidelberg, Germany) using the CellQuest software. For the detection of HLA-A2.1/IMP-specific CTL, cells were incubated with PE-labelled tetrameric MHC-I/IMP peptide complexes (tetramers) (Dunbar *et al.,* 1998) for 15 min at 37°C. Subsequently cells were counterstained with Tricolor-labelled CD8 specific mAb (Caltag, Burlingame, CA, USA) for 15 min on ice. After several washes 0.5 x 10$^6$ cells were analyzed by flow cytometry.

## 8. T cell stimulation assays:

For mixed lymphocyte reaction (allo-MLR), allogeneic CD4$^+$ or CD8$^+$ T cells were isolated by positive magnetic cell sorting according to the manufactures instructions (Miltenyi, Bergisch Gladbach, Germany). The T cells (2 x10$^5$/well) were cocultured with various numbers (220, 660, 2000, 6000) of DC in 96-well flat bottomed plates. After 4 days, cells were pulsed with 1 µCi/well [$^3$H]methyl-thymidine (Amersham Buchler, Braunschweig, Germany) and harvested 16 hours later onto glassfiber filters. Incorporation of [$^3$H]-thymidine was measured with a microplate counter (Wallac, Turku, Finland).

To assess the capability of infected DC to expand specific autologous cytotoxic T lymphocytes, DC were cocultured with T cells (DC : T ratio of 1:20) in 24-well plates in RPMI supplemented with 10 % heat-inactivated human serum. After seven days of culture without addition of exogenous cytokines the specificity and functionality of CTL was evaluated by staining with tetramers (P.R. Dunbar et al., Curr. Biol. 8, 413-416 (1998)) and by ELISPOT-assay.

9. Enzyme-linked immunospot assay (ELISPOT):

The ability of the transduced DC to stimulate antigen-specific CTL clones (i.e. IFN-( production) and the specificity of expanded autologous T cells for defined peptides was measured on a single cell basis using the ELISPOT technique. Highly protein-binding nitrocellulose membrane-bottomed 96-well plates (MAHA S4510, Millipore, Eschborn, Germany) were precoated with 0.1 $\mu$g/ml primary anti-IFN-( monoclonal antibody (1-D1k Mabtech, Stockholm, Sweden). After blocking with 5 % human serum 0.5-1x10$^4$ antigen-specific T cells were added per well. Subsequently 7.5x10$^4$ DC were carefully added. After 18 hoursof culture at 37°C the plates were washed six times with 0.05 % Tween in PBS and then incubated with biotinylated secondary anti-IFN-( monoclonal antibody (7-B6-1, Mabtech, Stockholm, Sweden). Plates were washed again six times and stained with streptavidin/peroxidase (Vectastain Elite kit, Vector Laboratories, Berlingame, CA; USA) and 3-amino-9-ethylcarbazole (AEC, Sigma, Deisenhofen, Germany) as substrate. Spots were counted using a special computer assisted video imaging analysis system (Carl Zeiss Vision, Eching, Germany), using the KS ELISPOT software version 4.1.143.

10. Expression of the MAGE-3 tumor antigen using recombinant influenza viruses:

Since influenza viruses can infect dendritic cells (DC) in an abortive way and at the same time enhance their T cell stimulatory potential (M. Cella et al., J. Exp. Med. 189, 821-829 (1999)), influenza virus derived vectors should be well suited to express antigens in these cells, which subsequently will be efficiently presented to the immune system. To introduce the tumor-associated antigen (TAA) MAGE-3 or, as a control, the green fluorescent protein (GFP) into influenza viruses, the RNA polymerase I (pol I) technique was applied (A. Zobel et al., Nucleic Acid Res. 21, 3607-3614 (1993)). To facilitate the detection of the protein, the MAGE-3 open reading frame was fused with the coding sequence for the AU-1 epitope tag. The coding sequences were inserted into the vector with the pol I promoter and viral packaging/promoter sequences (Fig.1). Due to the viral control sequences including the packaging signal, the pol I transcripts were incorporated into influenza virus particles when the transfected culture was inoculated with the avian influenza virus strain. Four to eight hours after infection, the cells started to stain green in UV light due to the expression of GFP (data not shown). The fraction of recombinants within the viral stock was determined by measuring their capacity to generate plaques and to express GFP in limiting dilution assays. Using the GFP recombinant virus as a model for the enrichment of the additional segment, we found that the heterologous segment is enriched from passage to passage. Starting from about 1-3 %, the content of recombinants in the viral stocks increased up to 30fold after two to three passages on MDCK cells (data not shown). This enrichment is due to the optimized viral promoter and packaging signal sequences present in the heterologous segment, which allow for a more efficient replication in comparison with the homologous influenza virus RNA segments (G. Neumann, G. Hobom, J. Gen. Virol. 76, 1709-1717 (1995)). To check for the presence of MAGE-3 recombinants in viral stocks, MDCK cells were inoculated with virus and MAGE-3 transcripts were detected from total cellular RNA by RT-PCR (Fig. 2A). To verify the MAGE-3 expression on the protein level, total protein extracts from infected MDCK cells were prepared. The heterologous protein was detected in immunoblots. As shown in Fig.2B high amounts of heterologous MAGE-3 protein could be detected. The protein expression of GFP was high resulting in a similar band intensity as MAGE-3 (Fig.2B). The recombinant viruses expressing GFP or MAGE-3 were termed FPV-GFP and FPV-MAGE3-AU1, respectively.

11. Efficient transfer of MAGE-3 into mature human DC using recombinant influenza virus:

To investigate whether heterologous genes can be transferred into human DC using recombinant influenza viruses, mature DC were generated. For this purpose CD14$^+$ monocytes were isolated from peripheral blood and treated with GM-CSF and IL-4 for six days; subsequently the cells were matured by the addition of IL-1$, IL-6, TNF-" and PGE$_2$. These cells showed the typical phenotype of fully mature DC (as described in Materials and Methods ). To determine whether human monocyte-derived DC are accessible to gene transfer by recombinant influenza virus, DC were infected with GFP expressing viruses at a MoI of 1. Microscopical examination revealed that almost all cells expressed the marker protein GFP (Fig. 3A). As expected the infection of these mature DC was abortive and did not result in cell lysis or production of progeny virus (data not shown). Quantitative analyses of green fluorescence using FACS showed that more than 90% of these DC were infected and expressed the heterologous protein (Fig. 3B). Maximal levels of GFP expression were detected within eight hours after infection. The protein was expressed for more than five days as was determined by daily examination of GFP fluorescence by microscopy. To verify the expression of the MAGE-3 gene, DC cultures were infected with the appropriate recombinant and in parallel with the GFP expressing virus. Protein extracts were subjected to immunoblot analyses. Both, the MAGE-3 gene and GFP gene were found to be expressed at high levels. For both proteins prominent specific bands of similar intensity could be demonstrated by using either an antibody binding the AU-1 tag of MAGE-3 or a GFP specific antiserum, respectively. (Fig. 4). Immature DC could be transduced with a similar efficiency (data not shown). However in the immature cell population infection with a MoI of 1 did result in increased cytophathic effects.

12. Phenotypical characterization of influenza virus infected DC:

To investigate whether infection with recombinant influenza virus affects the phenotype of DC, typical cell surface markers were analysed by FACS 24 hours after infection with the GFP-coding recombinants. As shown in Fig. 5, the transduced DC produced high levels of the typical surface markers. About 100% of the GFP-positive cells expressed CD86, CD 80, HLA class II and more than 80% of those cells HLA class I molecules. Other characteristic cell surface markers such as CD25, CD40 and CD1a were also detected on GFP[+] DC. Comparison with uninfected DC did not reveal any major differences in any of these markers. A modest down-modulation of CD83 surface expression was the only deviation observed (data not shown). The same expression patterns were obtained for DC infected with FPV-MAGE3-AU1 or wildtype virus. These data indicate that the infection with recombinant influenza virus and the high level expression of the heterologous antigens does not cause major phenotypic alterations in the infected DC .

13. T cell stimulation by influenza virus transduced DC:

The crucial point for the application of DC in immunotherapy is their capacity to stimulate both CD4[+] helper as well as CD8[+] cytotoxic T cells (CTL). In order to address this issue, we firstly tested the ability of the transduced DC to induce T cell proliferation in allogeneic MLR. Therefore, DC were infected with one of the recombinant influenza virus strains or with FPV wildtype and co-cultured for four days with allogeneic CD4[+] T cells. After a 16 hours pulse, the incorporation of [$^3$H]-thymidine was measured. As five individual experiments showed, all co-cultures resulted in a significant increase in [$^3$H]-thymidine incorporation, even than 300 and more T cells per DC were present. This indicated that DC infected with FPV-GFP or with FPV-MAGE3-AU1 or with FPV wildtype have an unimpaired high T cell stimulatory capacity (Fig.6). Similar results were obtained with allogeneic CD8[+] T cells (data not shown). Secondly, in order to evaluate the capacity of influenza virus transduced DC to present viral antigen to CD8[+] CTL, transduced DC were co-cultured with an influenza virus matrix protein (IMP) specific CTL clone and the activation was determined by IFN-( ELISPOT (T. Hanke et al., vaccine 16, 426-435 (1998)). As shown in three individual experiments, influenza virus transduced DC were potent stimulators of this T cell clone, indicating the presence of MHC-I/IMP complexes on its surface (Fig. 7). In contrast, uninfected DC with CTL or the CTL clone alone did not show any activation. Thirdly, we proved that these DC were capable to expand CD8[+] memory T cells in the absence of exogenously added cytokines; this is an unique property of DC. To this end, DC were infected with FPV-GFP or FPV-MAGE3-AU1. Uninfected DC and IMP peptide pulsed DC were prepared as negative and positive control, respectively. The DC were co-cultured with autologous CD8[+] T cells at a responder/stimulator ratio of 20:1. Specifically activated T cells were allowed to proliferate during a seven days culture period. Subsequently the number of IMP-specific CD8[+] cells were identified by double staining using monoclonal antibodies and tetrameric MHC-I/IMP complexes binding to specific T cell receptors (P.R. Dunbar et al., Curr. Biol. 8, 413-416 (1998)). As shown in Fig. 8A the infection with influenza virus recombinants resulted in an 11 to 18-fold increase of IMP specific CD8[+] positive T cells (for FPV-GFP: 0.49%, for FPV-MAGE: 0.32%; uninfected: 0.03%). Comparable numbers of specific CD8[+] T cells (0.57%) were obtained with IMP peptide pulsed DC . To assess whether the effector T cells expanded by the co-cultivation can be specifically activated, they were incubated with IMP peptide and analyzed for IFN-( production. As shown by ELISPOT assays, the number of individual IFN-(-producing T cells after co-culture with infected DC was about 30 times higher than that of the negative control (Fig. 8B). Next we investigated the ability of the FPV-MAGE3-AU1 transduced DC to generate MHC-I/MAGE-3 peptide complexes using a MAGE-3 specific CTL clone and ELISPOT as test system. As shown in Fig. 9, the transduced DC were able to stimulate IFN-( production from this CTL clone whereas the clone by itself did not spontaneously secrete IFN-(. Hence, TAA gene transfer by influenza virus vectors into DC yields antigen presenting cells, which present the corresponding tumor antigen apart from vector antigen and which specifically stimulate T cells.

**Claims**

1. A method for the expression of one or more tumour-associated antigens (TAA) or virus-associated antigens (VAA) by dendritic cells comprising

   (a) preparing a recombinant influenza virus containing a nucleotide sequence coding for the TAA or VAA, and
   (b) infecting dendritic cells with the recombinant influenza virus obtained in step (a).

2. The method of claim 1, wherein the nucleotide sequence coding for the TAA or VAA is present in the recombinant influenza virus in one of the regular segments or as an additional segment.

3. The method of claim 1 or 2, wherein the segment containing the nucleotide coding for the TAA or VAA contains terminal viral RNA sequences which have been modified by nucleotide substitutions in up to five positions, resulting in improved transcription rates of both the vRNA promoter as well as the cRNA promoter as present in the complementary sequences.

4. The method of claim 3, wherein the 12 nucleotide conserved influenza 3' terminal sequence has been modified by replacement of one to three nucleotides occurring in said sequence at positions 3, 5 and 8 relative to the 3' end by other nucleotides, and/or wherein the 13 nucleotide conserved influenza 5' terminal sequence has been modified by replacement of one or two nucleotides occurring in said sequence at positions 3 and 8 by other nucleotides.

5. The method of claim 4, wherein the replacements in the 3' terminal nucleotide sequence comprises the modifications G3A and C8U, or G3C and C8G.

6. The method of claim 4, wherein the replacements in the 3' terminal nucleotide sequence comprises the modifications G3A, U5C and C8U, or G3C, U5C and C8G.

7. The method of claim 5 or 6, which comprises a 3' terminal nucleotide sequence of (5')-CCUGUUUCUACU-3' or (5')-CCUGUUUUUACU-3'.

8. The method according to claims 4 to 7, wherein the 5' terminal nucleotide sequence comprises the modifications U3A and A8U resulting in a 5'-terminal sequence of 5'-AGAAGAAUCAAGG.

9. The method according to claims 1 to 8, wherein the recombinant influenza virus is a recombinant influenza A virus.

10. The method of one of claims 1 to 9, wherein

    (i) the TAA is selected from the class of cancer/testis antigens and preferably is MAGE-3, NY-ESO-1 or HOM-MEL-40;
    (ii) the VAA is selected from a Herpes simplex virus immediate early or early protein, a human papilloma virus-antigen, preferably $L_1$, $E_5$ or $E_6$, a Human Immunodeficiency Virus protein, preferably tat, rev or gag, or a Hepatitis C virus protein known to elicit T cell response in a mammal; or
    (iii) the TAA or VAA is a fusion protein, preferably comprising entirely or partially the proteins listed in (i) or (ii) above.

11. Dendritic cells expressing TAA or VAA which are obtainable according to the method of claims 1 to 10.

12. A pharmaceutical composition comprising dendritic cells of claim 11 or a mixture of different dendritic cells of claim 11, each expressing a different TAA or VAA.

13. Use of dendritic cells of claim 11 for preparing a medicament for tumour therapy or antiviral therapy.

14. A method for treating tumours or virus infections in a mammal comprising administering the mammal dendritic cells of claim 11.

19

Fig. 1

Fig. 2

β-ACTIN
GFP
β-ACTIN
MAGE-3-AU
β-ACTIN
MAGE-3-AU
GFP

MAGE-3-AU1
β-ACTIN
GFP

1,6 kb
1 kb
0,5 kb

**A** RT-PCR

FPV-MAGE-3-AU1
FPV-GFP
FPV-MAGE-3-AU1
FPV-GFP

— 66 kD

MAGE-3-AU1 ➤

— 46 kD

GFP ➤

— 30 kD

— 21,5 kD

αAU1          αGFP

**B** Immunoblot

Fig. 3

Fig. 4

Fig. 5

Fig. 6

Fig. 7

Fig. 8a

Fig. 8b

Fig. 9

**European Patent Office**

## PARTIAL EUROPEAN SEARCH REPORT

**Application Number**

which under Rule 45 of the European Patent Convention EP 00 12 3687 shall be considered, for the purposes of subsequent proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | RESTIFO N. P. ET AL: "Transfectant Influenza A viruses are effective recombinant immunogens in the treatment of experimental cancer" VIROLOGY, vol. 249, no. 1, 15 September 1998 (1998-09-15), pages 89-97, XP002170846 ORLANDO US | 1,2,9, 11-14 | C12N15/86 C07K14/47 C12N5/10 A61K48/00 A61K39/00 |
| Y | * the whole document * | 3-8,10 | |
| X | WO 00 53786 A (AZZEH MAYSA ;HOBOM GERD (DE); MENKE ANETTE (DE); ARTEMIS PHARMACEU) 14 September 2000 (2000-09-14) | 1,2,9, 11-14 | |
| Y | see specifically page 29 lines 14 to 17 and page 39 lines 13 to 21 * the whole document * | 3-8,10 | |
| | -/-- | | |

| | TECHNICAL FIELDS SEARCHED (Int.Cl.7) |
|---|---|
| | C12N C07K A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 29 June 2001 | Chambonnet, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

**INCOMPLETE SEARCH
SHEET C**

Application Number

EP 00 12 3687

Although claim 14 is directed to a method of treatment of the human/animal body (Article 52(4) EPC), the search has been carried out and based on the alleged effects of the composition.

---------------------

Claim(s) searched completely:
      1-13

Claim(s) not searched:
      14

Reason for the limitation of the search (non-patentable invention(s)):

Article 52 (4) EPC - Method for treatment of the human or animal body by therapy

**European Patent Office**

## PARTIAL EUROPEAN SEARCH REPORT

Application Number

EP 00 12 3687

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | STROBEL I. ET AL.: "Efficient transduction of mature human dendritic cells by using an avian influenza virus as a vector" THE JOURNAL OF INVESTIGATIVE DERMATOLOGY, vol. 110, no. 4, April 1998 (1998-04), page 605 XP001010009 | 1,2,9,11 | |
| Y | * the whole document * | 3-8,10, 12-14 | |
| X | STROBEL I. ET AL.: "Efficient transduction of mature human dendritic cells by using an avian influenza virus as a vector" JOURNAL OF DERMATOLOGICAL SCIENCE, vol. 16, no. Suppl. 1, March 1998 (1998-03), page S133 XP001009993 | 1,2,9,11 | TECHNICAL FIELDS SEARCHED (Int.Cl.7) |
| Y | * the whole document * | 3-8,10, 12-14 | |
| Y | EP 0 704 533 A (BAYER AG) 3 April 1996 (1996-04-03) * the whole document * | 3-8 | |
| Y | RUSSO V. ET AL.: "Dendritic cells acquire the MAGE-3 human tumor antigen from apoptotic cells and induce a class I-restricted T cell reponse" PROCEEDINGS OF THE NATIONAL ACADEMY OF SCIENCES OF USA, vol. 97, no. 5, 29 February 2000 (2000-02-29), pages 2185-2190, XP002170847 WASHINGTON US * the whole document * | 10 | |

-/--

European Patent
Office

## PARTIAL EUROPEAN SEARCH REPORT

Application Number

EP 00 12 3687

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| T | STROBEL I ET AL: "EFFICIENT EXPRESSION OF THE TUMOR-ASSOCIATED ANTIGEN MAGE-3 IN HUMAN DENDRITIC CELLS, USING AN AVIAN INFLUENZA VIRUS VECTOR" HUMAN GENE THERAPY, vol. 11, no. 16, 1 November 2000 (2000-11-01), pages 2207-2218, XP000986428 ISSN: 1043-0342 * the whole document * | 1-14 | |
| | | | TECHNICAL FIELDS SEARCHED (Int.Cl.7) |

EPO FORM 1503 03.82 (P04C10)

**ANNEX TO THE EUROPEAN SEARCH REPORT
ON EUROPEAN PATENT APPLICATION NO.**

EP 00 12 3687

This annex lists the patent family members relating to the patent documents cited in the above-mentioned European search report.
The members are as contained in the European Patent Office EDP file on
The European Patent Office is in no way liable for these particulars which are merely given for the purpose of information.

29-06-2001

| Patent document cited in search report | | Publication date | Patent family member(s) | | Publication date |
|---|---|---|---|---|---|
| WO 0053786 | A | 14-09-2000 | EP | 1035209 A | 13-09-2000 |
| | | | AU | 3427100 A | 28-09-2000 |
| EP 0704533 | A | 03-04-1996 | AU | 3607695 A | 26-04-1996 |
| | | | WO | 9610641 A | 11-04-1996 |
| | | | EP | 1091000 A | 11-04-2001 |
| | | | EP | 0783586 A | 16-07-1997 |
| | | | FI | 971272 A | 26-05-1997 |
| | | | JP | 2000509962 T | 08-08-2000 |
| | | | NZ | 293600 A | 28-01-1999 |

EPO FORM P0459

For more details about this annex : see Official Journal of the European Patent Office, No. 12/82